# EUROPEAN PATENT SPECIFICATION

(11) **EP 3 007 751 B1**
(45) Date of publication and mention of the grant of the patent: **11.12.2019**
(21) Application number: 14729024.1
(22) Date of filing: 11.06.2014
(51) Int. Cl.: A61M 5/42, A61M 5/20

(54) **SLIDING SLEEVE ATTACHMENT FOR AN INJECTION DEVICE**
SCHIEBEHÜLSENBEFESTIGUNG FÜR EINE INJEKTIONSVORRICHTUNG
ACCESSOIRE À MANCHON COULISSANT POUR UN DISPOSITIF D'INJECTION

(30) Priority: 11.06.2013 GB 201310393
(43) Date of publication of application: 20.04.2016
(73) Proprietor: Cilag GmbH International, 6300 Zug (CH)
(72) Inventor: BITAR, Ahmad, Hertfordshire SG8 7AJ (GB); JENNINGS, Douglas Ivan, Royston Hertfordshire SG8 9LJ (GB)
(74) Representative: Carpmaels & Ransford LLP
(86) International application number: PCT/EP2014/062162
(87) International publication number: WO 2014/198793

(56) References cited:
- EP-A1- 2 468 330
- WO-A2-2005/058393
- GB-A- 2 461 088
- US-A1- 2003 229 308
- US-A1- 2005 209 554
- US-A1- 2007 118 094

## Description

### Field of the invention

The present invention relates to an injection device that receives a syringe, extends the syringe and discharges its contents, commonly known as an auto-injector and a kit comprising the injection device.

### Background of the Invention

Auto-injectors are known from WO 95/35126 and EP-A-0 516 473 and tend to employ a drive spring and some form of release mechanism that releases the syringe from the influence of the drive spring once its contents are supposed to have been discharged, to allow it to be retracted by a return spring.

An auto-injector is known from WO 2007/036676 which has a locking mechanism which must be disengaged before the release mechanism can be activated. In its locked position, the locking mechanism also prevents forward movement of the syringe out of the injection device against the bias of the return spring, for example when a cap gripping a boot covering the syringe needle, is removed. In the injection device described in WO 2007/036676, the locking mechanism comprises a sleeve which protrudes from an open end of the injection device. The sleeve is biased into its extended position by a resilient spring mechanism which must be overcome to disengage the locking mechanism. The locking mechanism can be disengaged by, for example, moving the sliding sleeve inwardly into the injection device. This can be done by forcing the end of the sliding sleeve against tissue and then activating the release mechanism.

The documents WO2005/058393, GB2461088, EP2468330, and US2005/209554 disclose auto-injectors having different means to stabilize skin contact.

It can be difficult for a user to position the sliding sleeve at the correct angle against the tissue and maintain it in that position as the locking mechanism is disengaged. Ensuring the sliding sleeve is forced against tissue at the correct angle and held sufficiently stable on the tissue as the locking mechanism is overcome is important to ensure reliable operation of the device as it is activated.

### Summary of the invention

The auto-injector and kit of the present invention is designed to deal with the aforementioned problems.

The invention is set out in the appended set of claims.

In a first aspect of the invention, there is provided an auto-injector comprising a housing, including an exit aperture at a distal end of the auto-injector and a longitudinal axis, wherein the housing is adapted adapted to receive a syringe having a discharge nozzle, the syringe being moveable in the housing on actuation of the injection device along a longitudinal axis from a retracted position in which the discharge nozzle is contained within the housing and an extended position in which the discharge nozzle of the syringe extends from the housing through an exit aperture at a distal end of the device; a sliding component which extends, when in a first position, from the aperture, and is movable into the housing into a second, retracted, position, and wherein the sliding component comprises, at its distal end, a removable contact element adapted to be removably attached to the distal end of the sliding component, characterised in that the removable contact element comprises a cap configured to act as a suction element to secure the device to the skin.

Advantageously, the contact element is adapted for contact with the skin surface. The contact element provides an improved interface between the sliding component and the skin surface, as described further below. Since the contact element is removable, it can be retrofitted to existing injection devices, simplifying manufacture.

The injection device may further comprise an actuator and a drive adapted to be acted upon by the actuator and in turn act upon the syringe to advance it from its retracted position to its extended position and discharge its contents through the discharge nozzle.

The sliding component may be part of a locking mechanism moveable from an engaged position, when the sliding component is in its first position, to a disengaged position, when the sliding component is in its second position, and adapted to prevent actuation of the device when it is in its engaged position and permit actuation of the device when it is in its disengaged position.

The contact element may be adapted to adhere to the skin such that the end of sliding component is fixedly located on the skin. Adherence may be achieved in a number of ways, as described below.

The contact element comprises a cap, preferably a rubber cap, which is configured to act as a suction element to secure the device to the skin, thereby stabilising the injection device in the correct position for injection. The suction also acts to draw the skin into the contact element and towards the exit aperture. Pinching the skin in this way creates a suitable site for subcutaneous injections.

In addition, the contact element may comprise adhesive for adhering the contact element with the skin, such that the end of the sliding component is fixedly located on the skin. The adhesive secures the sliding component to the skin during operation of the device.

The contact element may have an inner and an outer diameter and a surface extending between the inner and the outer diameter and wherein the outer diameter is larger than the diameter of the sliding component.

The surface provides an improved contact area against tissue and acts to stabilise the sliding component as it pushed against a surface and moved into the second, retracted, position. The contact area may be configured to provide adherence to the skin, as described above, or simply have a larger surface area than the end of the sliding sleeve, so as to facilitate placement of the device on the skin. For example, a larger surface (and therefore a larger surface area in contact with the skin) may enable a user may to better discern whether the injection device is orientated correctly.

Advantageously, the inner diameter may be dimensioned for locating on the sliding component. This ensures the contact element can be readily fitted to the sliding component. In other words, the inner diameter is large enough and appropriately shaped to accommodate the sliding component. Preferably, the inner diameter is such that there is an interference fit between the sliding component and the contact element.

In addition the contact element may comprise at least one contact member having a surface for engaging a surface of the skin. The contact member contacts the skin, and provides an additional stabilising component for the contact element against the skin.

In addition the contact member may extend radially outwards from the longitudinal axis of the housing. This ensures that the device can be stabilised at the correct angle to the skin. As with the enlarged contact area described above, one or more contact elements may enable a user may to better discern whether the injection device is orientated correctly, due to the relatively large footprint compared with a conventional sliding sleeve.

In addition the contact element may comprise a plurality of contact members, increasing the stability that they provide. The more contact members are provided, the greater the footprint, and the better able the user will be to know that the injection device is correctly orientated.

In a particularly preferred embodiment, the contact element may additionally comprise three contact members, spaced equidistantly from each other. This creates a tripod effect which is effective in stabilising the sliding component when it is pressed onto a surface.

In addition the contact member may comprise a second surface for engaging a surface of the housing. When the sliding component moves into its retracted position, the second surface engages the housing providing visual and tactile feedback that the sliding component has been retracted.

The sliding component may comprise a sliding sleeve, which acts as part of a locking mechanism to prevent the device being actuated and, once in it is second retracted position, acts to disengage a locking mechanism to allow an injection to be carried out.

The contact element may be disposed on the distal end of the sliding sleeve.

The distal end of the sliding sleeve is that which contacts tissue. Therefore, for optimum stabilising effect, the contact should be disposed at the distal end of the sliding sleeve.

The contact element may disposable. Since the injection devices to which the contact element is applied may be single use devices, it is useful that the contact element be similarly disposable.

In a second aspect of the invention, there is provided a kit comprising an auto-injector comprising a housing, including an exit aperture at the distal end of the auto-injector and a longitudinal axis, wherein the housing is adapted to receive a syringe having a discharge nozzle, the syringe being moveable in the housing on actuation of the auto-injector along a longitudinal axis from a retracted position in which the discharge nozzle is contained within the housing and an extended position in which the discharge nozzle of the syringe extends from the housing through an exit aperture at a distal end of the device, a sliding component which extends, when in a first position, from the aperture, and is movable into the housing into a second, retracted, position; and a contact element adapted to be removably attached to the distal end of the sliding component, characterised in that the removable contact element comprises a cap configured to act as a suction element to secure the device to the skin.

In any embodiment, the injection device may contain a substance selected from the group consisting of: golimumab, hormones, antitoxins, substances for the control of pain, substances for the control of thrombosis, substances for the control or elimination of infection, peptides, proteins, human insulin or a human insulin analogue or derivative, polysaccharide, DNA, RNA, enzymes, antibodies, oligonucleotide, antiallergics, antihistamines, anti-inflammatories, corticosteroids, disease modifying anti-rheumatic drugs, erythropoietin, or vaccines, for use in the treatment or prevention of rheumatoid arthritis, psoriatic arthritis, ankylosing spondylitis, ulcerative colitis, hormone deficiency, toxicity, pain, thrombosis, infection, diabetes mellitus, diabetic retinopathy, acute coronary syndrome, angina, myocardial infarction, atherosclerosis, cancer, macular degeneration, allergy, hay fever, inflammation, anaemia, or myelodysplasia, or in the expression of protective immunity.

By 'the injection device may contain a substance' it is meant that the substance may be contained within a suitable medicament container, such as a vial or syringe, within the injection device. Such medicament container may contain other substances, such as further active or inactive ingredients.

In a further aspect of the invention, a substance is provided, the substance being selected from the group consisting of: golimumab, hormones, antitoxins, substances for the control of pain, substances for the control of thrombosis, substances for the control or elimination of infection, peptides, proteins, human insulin or a human insulin analogue or derivative, polysaccharide, DNA, RNA, enzymes, antibodies, oligonucleotide, antiallergics, antihistamines, anti-inflammatories, corticosteroids, disease modifying anti-rheumatic drugs, erythropoietin, or vaccines, for use in the treatment or prevention of rheumatoid arthritis, psoriatic arthritis, ankylosing spondylitis, ulcerative colitis, hormone deficiency, toxicity, pain, thrombosis, infection, diabetes mellitus, diabetic retinopathy, acute coronary syndrome, angina, myocardial infarction, atherosclerosis, cancer, macular degeneration, allergy, hay fever, inflammation, anaemia, or myelodysplasia, or in the expression of protective immunity, by delivery of said substance to a human subject using an injection device according to any of the above embodiments.

In yet another aspect of the invention, an injection device is provided for use in the treatment or prevention of rheumatoid arthritis, psoriatic arthritis, ankylosing spondylitis, ulcerative colitis, hormone deficiency, toxicity, pain, thrombosis, infection, diabetes mellitus, diabetic retinopathy, acute coronary syndrome, angina, myocardial infarction, atherosclerosis, cancer, macular degeneration, allergy, hay fever, inflammation, anaemia, or myelodysplasia, or in the expression of protective immunity, by delivery of a substance selected from the group consisting of: golimumab, hormones, antitoxins, substances for the control of pain, substances for the control of thrombosis, substances for the control or elimination of infection, peptides, proteins, human insulin or a human insulin analogue or derivative, polysaccharide, DNA, RNA, enzymes, antibodies, oligonucleotide, antiallergics, antihistamines, anti-inflammatories, corticosteroids, disease modifying anti-rheumatic drugs, erythropoietin, or vaccines, to a human subject by using the injection device, where the injection device is an injection device of any of the above embodiments.

By 'delivery of a substance' it is meant that the injection device is used to inject said substance into the human subject, for example by subcutaneous, intradermal or intramuscular injection. Said substance may be administered in combination with other substances, such as further active or inactive ingredients.

### Brief Description of the Drawings

The present invention is now described by way of example with reference to the accompanying drawings, in which:-
Figure 1 shows a perspective view of an injection device having a locking mechanism including a sliding sleeve;
Figure 2 shows a cutaway side view of an injection device having a locking mechanism including a sliding sleeve;
Figure 3 show a side view of an injection device having a locking mechanism including a sliding sleeve;
Figure 4a shows a side view of a contact element according to the present invention attached to an injection device;
Figure 4b shows a perspective view of the contact element of figure 4a;
Figure 4c shows a plan view of an alternative contact element according to the present invention attached to an injection device;
Figure 4d shows a perspective view of the contact element of figure 4c;
Figure 5a shows a side view of an alternative contact element in accordance with the present invention attached to an injection device;
Figure 5b shows a perspective view of the contact element of figure 5a;
Figure 6a shows a side view of an alternative contact element in accordance with the present invention attached to the sliding component;
Figure 6b shows a perspective view of the contact element of figure 6a;
Figure 7a shows a plan view of an alternative contact element in accordance with the present invention; and
Figure 7b shows a perspective view of the contact element of figure 7a attached to an injection device.

### Detailed description of the drawings

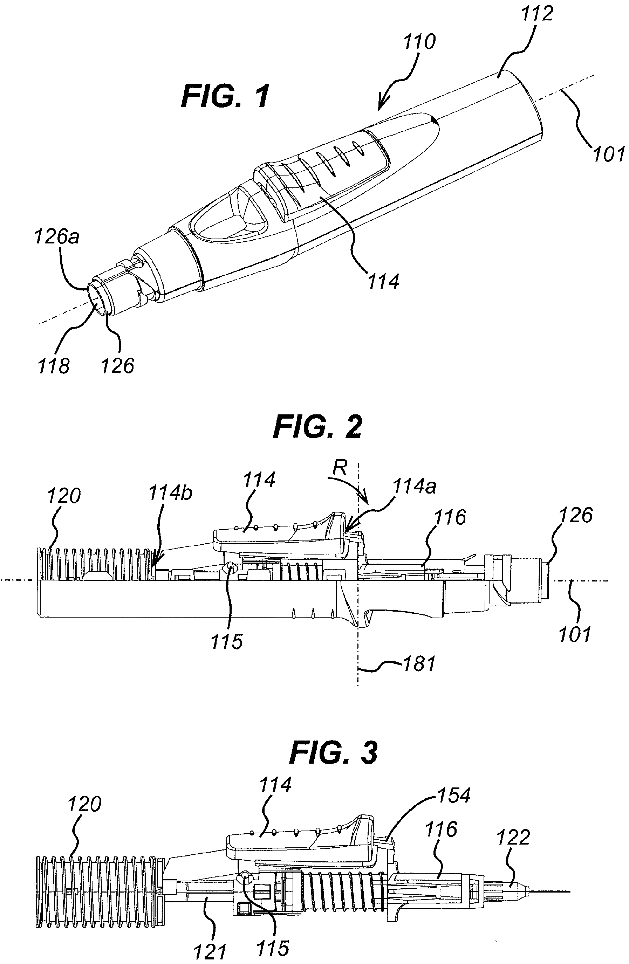

Figures 1 to 3 show an injection device 110. The injection device 110 has an injection device housing 112 and a longitudinal axis 101.

A syringe (figure 3) is contained in the housing 112. The injection device 110 comprises trigger 114 (actuator) and a releasable locking mechanism 116. The trigger 114 has a first end 114a and a second end 114b. The trigger 114 is rotatable about a pivot 115 from a rest position (as shown in Figure 2) to an active position. The second end 114b of the trigger 114 connects with a drive coupling 121 which is acted upon by a drive spring 120. The drive coupling 121 is in communication with the syringe 122.

Rotation of the trigger 114 about the pivot 115 in a direction R (i.e. downwards into the housing 112 at its first end 114a) causes the second end 114b of the trigger 114 to disengage from the drive coupling 121, thereby letting the drive spring 120 drive the syringe 122 (via the drive coupling 121) along the longitudinal axis 101 and out of an aperture 118 in the housing 112.

The releasable locking mechanism 116 is in communication with sliding sleeve 126 which protrudes, when in a first position, from the aperture 118 in the housing 112. The locking mechanism 116 is deactivated by movement of the sliding sleeve 126 along the longitudinal axis 101 into the housing 112 into a second position.

A first end 126a of the sliding sleeve 126 can be placed against a body into which drug is being delivered, thereby deactivating the releasable locking mechanism 116 and allowing the trigger 114 to rotate in direction R from its rest position to its active position.

The trigger 114 is provided at its first end 114a with a first portion having a cut-out. The first portion extends from the first end 114a of the trigger 114a in a direction substantially parallel to the longitudinal axis 101.

The releasable locking mechanism 116 includes a protrusion 154 which projects in a direction along a perpendicular axis 181 which is perpendicular to the longitudinal axis 101. The cut-out is dimensioned to receive the protrusion.

When the releasable locking mechanism 116 is in its first position, an end of the protrusion abuts an under-surface of the first portion 150, thereby preventing rotation of the trigger 114.

When the releasable locking mechanism 116 is in its second position (not shown) following movement of the sliding sleeve 126 into the housing 112, the cut-out is positioned above the end of the protrusion 154 allowing it to pass over the protrusion when a downwards force is applied the trigger 114. Hence, the trigger 114 is no longer prevented from rotating and disengages itself from the drive coupling 121, thereby extending the syringe.

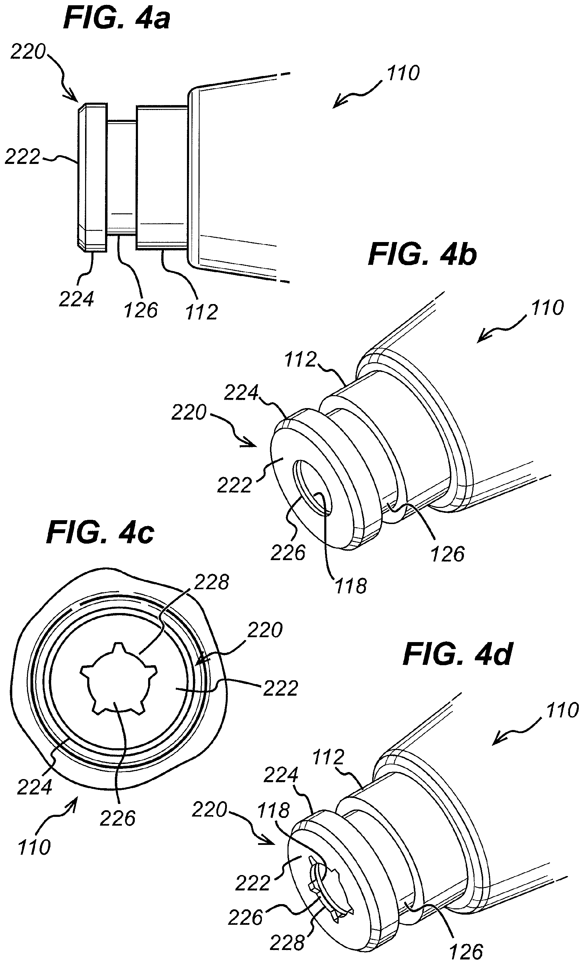

Figures 4a and 4b show a contact element 220 disposed on the distal end of sliding sleeve 126. The contact element is an additional component which acts as a flange when it is connected to the distal end of the sliding sleeve. The component is a discrete component which is removable from the device. Thus, it can more readily be retrofitted to existing injection devices either at the point of manufacture or by a user. The component has an aperture 226 which is placed over the aperture 118 of the housing 112 through which the discharge nozzle extends, and allows the discharge nozzle to extend therethrough. The outer diameter 224 of the component is larger than the diameter of the sliding sleeve 126 and the component has a tissue contacting surface 222 extending from the aperture to the outer diameter 224 which, when the contact element 220 is affixed to the distal end of the sliding sleeve 126 faces away from the distal end of the housing 112.

In use, the tissue contacting surface 222 of the contact element 220 is placed against the skin of the user and used as a stabilising surface to firstly correctly position the injection device 110 at the correct angle relative to the user's skin, and secondly, to ensure the injection device 110 remains stable as it is pressed towards the housing 112 to move the sliding sleeve 126 into the second, retracted position. The sliding sleeve 126 uses the contact element 220 to push against the skin as it is moved into its second position. The surface of the component opposite to the tissue contacting surface may be adapted to engage the housing. In this embodiment, when the sliding sleeve is moved into its retracted position, the housing engages the opposite surface of the additional component to provide the user with a visual and tactile indication that the sliding sleeve is in the retracted position.

In an alternative embodiment, shown in figure 4c, the inner diameter of the contact element may be adapted so that it grips the skin as it is pressed onto it. For example, as shown in figure 4c, the inner diameter of the contact element, i.e. that diameter which surrounds the aperture 226, is castellated so as produce resilient protrusions 228 into the aperture 226. The protrusions 228 flex as the contact element is placed on the skin and then return to the initial position to pinch the skin between the protrusions 228. Alternatively, the contact element may comprise a portion of resilient material which allows the aperture 226 of the contact element 220 to expand as it is placed in the user's skin and then contract to fix the device in place about the user's skin. This resilience creates a more secure connection between the stabilising contact element and the user's skin.

Any of the contact elements 220 shown in figures 4a to 4c may be made of rubber or TPE for improved tactile feel and grip.

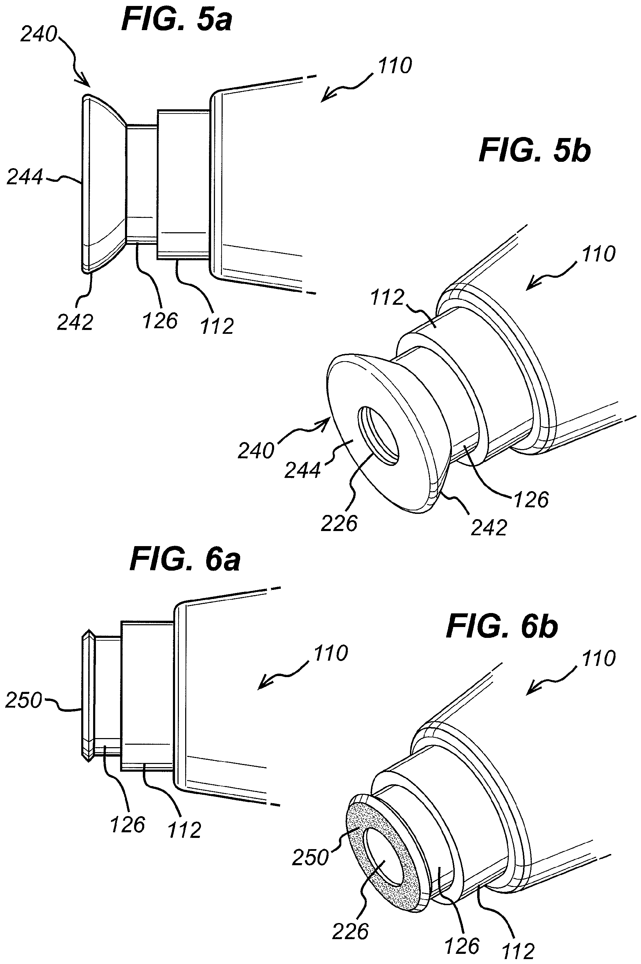

Figures 5a and 5b show an alternative contact element 240 in the form of a cap 242 which acts as a suction element. As shown in figure 5, the cap is positioned at the distal end of the sliding sleeve 126. The cap may be formed of a rubber material or any material, such as TPE, which is resiliently deformable as the cap is placed on the skin. These materials also provide improved tactile feel and grip.

In use the tissue contacting surface of the cap 242 is placed against the skin such that it adheres to the skin. Due to its resilient nature, the cap 242 acts both to adhere the sliding sleeve 126 to the skin and to pinch the skin by acting as a suction element and creating a vacuum between its contact surface 244 and the skin. This draws and compresses the skin towards the aperture 118 from which the discharge nozzle extends, which is desirable when the medication is to be injected subcutaneously.

Figures 6 and 6b show an alternative embodiment in which adhesive surface 250 is positioned on the distal end of a sliding sleeve 126, and used to adhere the distal end of the sliding sleeve 126 to the skin. When the distal end of the sliding sleeve 126 is correctly positioned on the skin, the adhesive surface holds the sliding sleeve 126 in that position so that the injection device 110 is oriented at the correct angle for operation and the sliding sleeve 126 can be moved into its retracted position correctly. Adhesive can be applied to any of the contact surfaces 222, 244 of the alternative embodiments to assist in securing the injection device 110 to the skin.

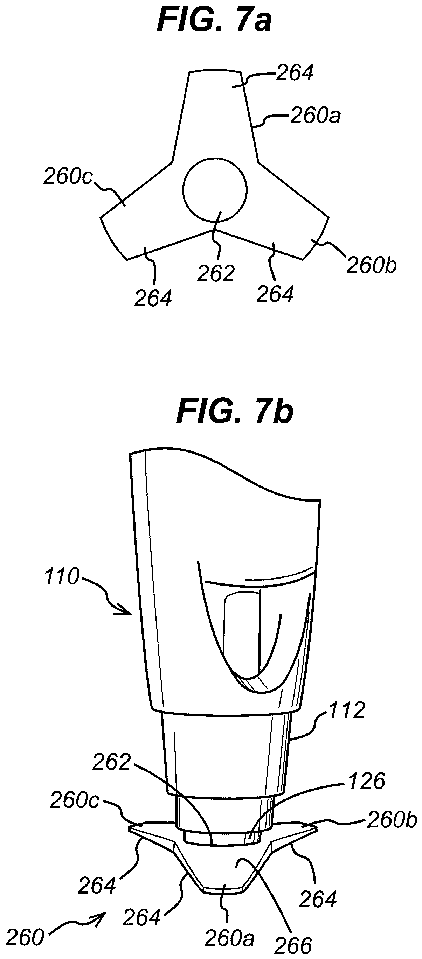

Figures 7a and 7b show an alternative contact element 260 which acts to stabilise the device on the skin. The contact element shown in this figure comprises three contact members in the form of protrusions 260a, 260b, 260c which are arranged about an aperture 262. The number of protrusions provided may differ. For example, two, four or five protrusions may be provided. The aperture 262 is affixed to the sliding sleeve 126, as shown in figure 7b, so that it aligns with the aperture 118 of the housing 112 through which the discharge nozzle extends. The protrusions 260a, 260b, 260c are arranged at equidistant angles around the aperture 262 and comprise skin contacting surfaces 264. When the contact element 260 is positioned on the sliding sleeve 126, the skin contacting surfaces 264 face away from the sliding sleeve 126. The contact element 260 may be formed of rubber or TPE for improved tactile feel and grip. In operation, the skin contacting surfaces 264 are positioned on the user's skin and the protrusions 260a, 260b, 260c used to stabilise the device on the user's skin. Once the injection device 110 is pushed towards the user's skin, and the sliding sleeve 126 is in its retracted position, the distal end of the housing 112 contacts the surface of the protrusions 260a, 260b, 260c opposite the skin contacting surface providing a visual and tactile indication to the user that the sliding sleeve 126 is in its retracted position and that the locking mechanism has been unlocked, readying the injection device 110 for actuation.

A kit may be supplied which includes an injection device with the contact element pre-attached or it may be supplied with a separate contact element which is attached by the user prior to injection and once a protective cap is removed.

In use, such an injection device as described above might be used to deliver substances such as: golimumab, hormones, antitoxins, substances for the control of pain, substances for the control of thrombosis, substances for the control or elimination of infection, peptides, proteins, human insulin or a human insulin analogue or derivative, polysaccharide, DNA, RNA, enzymes, antibodies, oligonucleotide, antiallergics, antihistamines, anti-inflammatories, corticosteroids, disease modifying anti-rheumatic drugs, erythropoietin, or vaccines, for use in the treatment or prevention of rheumatoid arthritis, psoriatic arthritis, ankylosing spondylitis, ulcerative colitis, hormone deficiency, toxicity, pain, thrombosis, infection, diabetes mellitus, diabetic retinopathy, acute coronary syndrome, angina, myocardial infarction, atherosclerosis, cancer, macular degeneration, allergy, hay fever, inflammation, anaemia, or myelodysplasia, or in the expression of protective immunity. In addition to these substances, any medicament contained within the injection device may also include other substances, such as inactive ingredients, as a skilled person would appreciate.

It will of course be understood by the person skilled in the art that particular substances are efficacious for use in the treatment or prevention of particular conditions, as is well known in the art. For instance, it is known that antiallergics are efficacious for use in the treatment or prevention of allergies; antihistamines are efficacious for use in the treatment or prevention of hay fever; anti-inflammatories are efficacious for use in the treatment or prevention of inflammation; and so on. Accordingly, any selection of one or more substances listed herein or in the claims for use in the treatment or prevention of one or more conditions for which those substance(s) are known to be efficacious is envisaged.

In a particular example, however, golimumab is known to be efficacious for use in the treatment or prevention of one or more of rheumatoid arthritis, psoriatic arthritis, ankylosing spondylitis or ulcerative colitis, or any combination of rheumatoid arthritis, psoriatic arthritis, ankylosing spondylitis and ulcerative colitis, or all of rheumatoid arthritis, psoriatic arthritis, ankylosing spondylitis and ulcerative colitis.

Golimumab may optionally be used in combination with one or more inactive ingredients such as any or all of L-histidine, L-histidine monohydrochloride monohydrate, sorbitol, polysorbate 80, and water. Golimumab may present in a composition in which golimumab is the only active ingredient. For example, golimumab may administered as SIMPONI®.

It will of course be understood that the present invention has been described above purely by way of example and modifications of detail can be made within the scope of the invention as defined in the claims.

## Claims

1. An auto-injector (110) comprising:
a housing (112), including an exit aperture at a distal end of the auto-injector and a longitudinal axis (101);
wherein the housing is adapted to receive a syringe (122) having a discharge nozzle, the syringe being moveable in the housing on actuation of the auto-injector along the longitudinal axis (101) from a retracted position in which the discharge nozzle is contained within the housing and an extended position in which the discharge nozzle of the syringe extends from the housing through the exit aperture (118) at the distal end of the auto-injector;
a sliding component (126) which extends, when in a first position, from the aperture, and is movable into the housing into a second, retracted, position; and
a removable contact element (220) adapted to be removably attached to the distal end of the sliding component, **characterised in that** the removable contact element comprises a cap configured to act as a suction element to secure the device to the skin.

2. The auto-injector according to claim 1, further comprising:
an actuator (114); and
a drive (121) adapted to be acted upon by the actuator and in turn act upon the syringe to advance it from its retracted position to its extended position and discharge its contents through the discharge nozzle.

3. The auto-injector according to claim 1 or claim 2, wherein the sliding component is part of a locking mechanism (116) moveable from an engaged position, when the sliding component is in its first position, to a disengaged position, when the sliding component is in its second position, and adapted to prevent actuation of the device when it is in its engaged position and permit actuation of the device when it is in its disengaged position.

4. The auto-injector according to any preceding claim, wherein the contact element is configured to draw the user's skin towards the exit aperture.

5. The auto-injector according to claim 4, wherein the contact element comprises a resilient portion (228) for adhering the contact element with the skin such that the end of sliding component is fixedly located on the skin.

6. The auto-injector according to claim 5, wherein the contact element comprises two resiliently biased arms.

7. The auto-injector according to any one of claims 1 to 4, wherein the contact element comprises a flexible cap (242) for adhering the contact element with the skin such that the end of sliding component is fixedly located on the skin.

8. The auto-injector according to any one of claims 1 to 3, wherein the contact element comprises adhesive (250) for adhering the contact element with the skin, such that the end of the sliding component is fixedly located on the skin.

9. The auto-injector according to any one of claims 1 to 3, wherein the contact element has an inner and an outer diameter (224) and a surface extending between the inner and the outer diameter and wherein the outer diameter is larger than the diameter of the sliding component.

10. The auto-injector according to claim 9, wherein the inner diameter is dimensioned for locating on the sliding component.

11. The auto-injector according to any one of claims 1 to 3, wherein the contact element comprises at least one contact member (260a) having a surface for engaging a surface of the skin, and which preferably extends radially from the longitudinal axis of the housing.

12. The auto-injector of claim 11, wherein the contact element comprises a plurality of contact members, preferably three contact members, spaced equidistantly from each other.

13. The auto-injector of claim 11 or claim 12 wherein the contact member comprises a second surface for engaging a surface of the housing.

14. The auto-injector of any preceding claim, wherein the sliding component comprises a sliding sleeve and wherein the contact element is disposed on the distal end of the sliding sleeve.

15. The auto-injector of any preceding claim, wherein the contact element is disposable.

16. A kit comprising:
an auto-injector (110) comprising:
a housing (112), including an exit aperture at a distal end of the auto-injector and a longitudinal axis (101);
wherein the housing is adapted to receive a syringe (122) having a discharge nozzle, the syringe being moveable in the housing on actuation of the auto-injector along the longitudinal axis (101) from a retracted position in which the discharge nozzle is contained within the housing and an extended position in which the discharge nozzle of the syringe extends from the housing through the exit aperture (118) at the distal end of the auto-injector;
a sliding component (126) which extends, when in a first position, from the aperture, and is movable towards the housing into a second, retracted, position; and
a contact element (220) adapted to be removably attached to the distal end of the sliding component, **characterised in that** the contact element comprises a cap configured to act as a suction element to secure the device to the skin.

## Patentansprüche

1. Autoinjektor (110), umfassend:
ein Gehäuse (112), das eine Austrittsöffnung an einem distalen Ende des Autoinjektors und eine Längsachse (101) aufweist;
wobei das Gehäuse zur Aufnahme einer Spritze (122) mit einer Auslassdüse angepasst ist, wobei die Spritze im Gehäuse bei Betätigung des Autoinjektors entlang der Längsachse (101) von einer eingefahrenen Stellung, in der die Auslassdüse im Gehäuse enthalten ist, und einer ausgefahrenen Stellung, in der die Auslassdüse der Spritze sich vom Gehäuse durch die Austrittsöffnung (118) am distalen Ende des Autoinjektors erstreckt, bewegbar ist;
eine Gleitkomponente (126), die sich in einer ersten Stellung von der Öffnung erstreckt und in das Gehäuse in eine zweite eingefahrene Stellung bewegbar ist; und
ein entfernbares Kontaktelement (220), das angepasst ist, entfernbar am distalen Ende der Gleitkomponente befestigt zu werden, **dadurch gekennzeichnet, dass** das entfernbare Kontaktelement einen Kappe umfasst, die dazu ausgelegt ist, als ein Saugelement zur Befestigung der Vorrichtung an der Haut zu dienen.

2. Autoinjektor nach Anspruch 1, ferner umfassend:
ein Betätigungselement (114); und
einen Antrieb (121), der angepasst ist, dass das Betätigungselement auf ihn einwirkt und wiederum auf die Spritze einzuwirken, um diese aus ihrer eingefahrenen Stellung in ihre ausgefahrene Stellung vorzuschieben und ihren Inhalt durch die Auslassdüse abzugeben.

3. Autoinjektor nach Anspruch 1 oder Anspruch 2, wobei die Gleitkomponente Teil eines Sperrmechanismus (116) ist, der aus einer Eingriffsstellung, in der sich die Gleitkomponente in ihrer ersten Stellung befindet, in eine nicht eingegriffene Stellung, in der sich die Gleitkomponente in ihrer zweiten Stellung befindet, bewegbar ist und angepasst ist, in der Eingriffsstellung eine Betätigung der Vorrichtung zu verhindern, und in der nicht eingegriffenen Stellung die Betätigung der Vorrichtung zu erlauben.

4. Autoinjektor nach einem der vorhergehenden Ansprüche, wobei das Kontaktelement dazu ausgelegt ist, die Haut des Benutzers zur Austrittsöffnung zu ziehen.

5. Autoinjektor nach Anspruch 4, wobei das Kontaktelement einen nachgiebigen Abschnitt (228) zum Anhaften des Kontaktelements an der Haut umfasst, so dass das Ende der Gleitkomponente fest auf der Haut angeordnet ist.

6. Autoinjektor nach Anspruch 5, wobei das Kontaktelement zwei elastisch vorgespannte Arme umfasst.

7. Autoinjektor nach einem der Ansprüche 1 bis 4, wobei das Kontaktelement eine flexible Kappe (242) zum Anhaften des Kontaktelements an der Haut umfasst, so dass das Ende der Gleitkomponente fest auf der Haut angeordnet ist.

8. Autoinjektor nach einem der Ansprüche 1 bis 3, wobei das Kontaktelement einen Klebstoff (250) zum Anhaften des Kontaktelements an der Haut umfasst, so dass das Ende der Gleitkomponente fest auf der Haut angeordnet ist.

9. Autoinjektor nach einem der Ansprüche 1 bis 3, wobei das Kontaktelement einen Innen- und einen Außendurchmesser (224) und eine sich zwischen dem Innen- und dem Außendurchmesser erstreckende Oberfläche aufweist und wobei der Außendurchmesser größer als der Durchmesser der Gleitkomponente ist.

10. Autoinjektor nach Anspruch 9, wobei der Innendurchmesser zur Platzierung auf der Gleitkomponente dimensioniert ist.

11. Autoinjektor nach einem der Ansprüche 1 bis 3, wobei das Kontaktelement mindestens ein Kontaktelement (260a) mit einer Oberfläche für einen Eingriff mit einer Hautoberfläche umfasst, und die sich vorzugsweise radial von der Längsachse des Gehäuses erstreckt.

12. Autoinjektor nach Anspruch 11, wobei das Kontaktelement eine Vielzahl von Kontaktelementen umfasst, vorzugsweise drei Kontaktelemente, die im gleichen Abstand voneinander angeordnet sind.

13. Autoinjektor nach Anspruch 11 oder Anspruch 12, wobei das Kontaktelement eine zweite Oberfläche für einen Eingriff mit einer Oberfläche des Gehäuses umfasst.

14. Autoinjektor nach einem der vorhergehenden Ansprüche, wobei die Gleitkomponente eine Gleithülse umfasst und wobei das Kontaktelement am distalen Ende der Gleithülse angeordnet ist.

15. Autoinjektor nach einem der vorhergehenden Ansprüche, wobei das Kontaktelement ein Einwegartikel ist.

16. Kit, umfassend:
Autoinjektor (110), umfassend:
ein Gehäuse (112), das eine Austrittsöffnung an einem distalen Ende des Autoinjektors und eine Längsachse (101) aufweist;
wobei das Gehäuse zur Aufnahme einer Spritze (122) mit einer Auslassdüse angepasst ist, wobei die Spritze im Gehäuse bei Betätigung des Autoinjektors entlang der Längsachse (101) von einer eingefahrenen Stellung, in der die Auslassdüse im Gehäuse enthalten ist, in eine ausgefahrene Stellung, in der die Auslassdüse der Spritze sich vom Gehäuse durch die Austrittsöffnung (118) am distalen Ende des Autoinjektors erstreckt, bewegbar ist;
eine Gleitkomponente (126), die sich in einer ersten Stellung von der Öffnung erstreckt und zum Gehäuse in eine zweite eingefahrene Stellung bewegbar ist; und
ein Kontaktelement (220), das angepasst ist, entfernbar am distalen Ende der Gleitkomponente befestigt zu werden, **dadurch gekennzeichnet, dass** das Kontaktelement einen Kappe umfasst, die dazu ausgelegt ist, als ein Saugelement zur Befestigung der Vorrichtung an der Haut zu dienen.

## Revendications

1. Auto-injecteur (110) comprenant :
un logement (112), comprenant une ouverture de sortie au niveau d'une extrémité distale de l'auto-injecteur et un axe longitudinal (101) ;
le logement étant conçu pour recevoir une seringue (122) possédant une buse d'évacuation, la seringue étant mobile dans le logement lors de l'actionnement de l'auto-injecteur le long de l'axe longitudinal (101) depuis une position rétractée dans laquelle la buse d'évacuation est contenue dans le logement et une position étendue dans laquelle la buse d'évacuation de la seringue s'étend depuis le logement à travers l'ouverture de sortie (118) au niveau de l'extrémité distale de l'auto-injecteur ;
un composant coulissant (126) qui s'étend, lorsqu'il est dans une première position, depuis l'ouverture, et qui est mobile vers le logement dans une seconde position rétractée ; et
un élément de contact amovible (220) conçu pour être fixé de manière amovible à l'extrémité distale du composant coulissant, **caractérisé en ce que** l'élément de contact amovible comprend un bouchon conçu pour faire office d'élément d'aspiration afin de fixer solidement le dispositif à la peau.

2. Auto-injecteur selon la revendication 1, comportant en outre :
un actionneur (114) ; et
un entraînement (121) conçu pour être soumis à l'action de l'actionneur et à son tour pour agir sur la seringue pour la faire avancer de sa position rétractée à sa position étendue et pour évacuer son contenu par la buse d'évacuation.

3. Auto-injecteur selon selon la revendication 1 ou la revendication 2, dans lequel le composant coulissant fait partie d'un mécanisme de verrouillage (116) mobile depuis une position en prise, lorsque le composant coulissant est dans sa première position, vers une position libérée, lorsque le composant coulissant est dans sa seconde position, et conçu pour empêcher l'actionnement du dispositif lorsqu'il est dans sa position en prise et pour permettre l'actionnement du dispositif lorsqu'il est dans sa position libérée.

4. Auto-injecteur selon l'une quelconque des revendications précédentes, dans lequel l'élément de contact est conçu pour aspirer la peau de l'utilisateur vers l'ouverture de sortie.

5. Auto-injecteur selon la revendication 4, dans lequel l'élément de contact comprend une partie résiliente (228) permettant de faire adhérer l'élément de contact à la peau de sorte que l'extrémité du composant coulissant soit située à demeure sur la peau.

6. Auto-injecteur selon la revendication 5, dans lequel l'élément de contact comprend deux bras sollicités de manière résiliente.

7. Auto-injecteur selon l'une quelconque des revendications 1 à 4, dans lequel l'élément de contact comprend un bouchon flexible (242) permettant de faire adhérer l'élément de contact à la peau de sorte que l'extrémité du composant coulissant soit située à demeure sur la peau.

8. Auto-injecteur selon l'une quelconque des revendications 1 à 3, dans lequel l'élément de contact comprend un adhésif (250) permettant de faire adhérer l'élément de contact à la peau, de sorte que l'extrémité du composant coulissant soit située à demeure sur la peau.

9. Auto-injecteur selon l'une quelconque des revendications 1 à 3, dans lequel l'élément de contact a un diamètre interne et un diamètre externe (224) et une surface s'étendant entre le diamètre interne et le diamètre externe et dans lequel le diamètre externe est plus grand que le diamètre du composant coulissant.

10. Auto-injecteur selon la revendication 9, dans lequel le diamètre interne est dimensionné pour se trouver sur le composant coulissant.

11. Auto-injecteur selon l'une quelconque des revendications 1 à 3, dans lequel l'élément de contact comprend au moins un élément de contact (260a) possédant une surface pour entrer en prise avec une surface de la peau, et qui s'étend de préférence radialement depuis l'axe longitudinal du logement.

12. Auto-injecteur selon la revendication 11, dans lequel l'élément de contact comprend une pluralité d'éléments de contact, de préférence trois éléments de contact, à égale distance les uns des autres.

13. Auto-injecteur selon la revendication 11 ou la revendication 12 dans lequel l'élément de contact comprend une seconde surface pour entrer en prise avec une surface du logement.

14. Auto-injecteur selon l'une quelconque des revendications précédentes, dans lequel le composant coulissant comprend un manchon coulissant et dans lequel l'élément de contact est disposé sur l'extrémité distale du manchon coulissant.

15. Auto-injecteur selon l'une quelconque des revendications précédentes, dans lequel l'élément de contact est jetable.

16. Kit comprenant :
un auto-injecteur (110) comprenant :
un logement (112), comprenant une ouverture de sortie au niveau d'une extrémité distale de l'auto-injecteur et un axe longitudinal (101) ;
le logement étant conçu pour recevoir une seringue (122) possédant une buse d'évacuation, la seringue étant mobile dans le logement lors de l'actionnement de l'auto-injecteur le long de l'axe longitudinal (101) depuis une position rétractée dans laquelle la buse d'évacuation est contenue dans le logement et une position étendue dans laquelle la buse d'évacuation de la seringue s'étend depuis le logement à travers l'ouverture de sortie (118) au niveau de l'extrémité distale de l'auto-injecteur ;
un composant coulissant (126) qui s'étend, lorsqu'il est dans une première position, depuis l'ouverture, et qui est mobile en direction du logement dans une seconde position rétractée ; et
un élément de contact (220) conçu pour être fixé de manière amovible à l'extrémité distale du composant coulissant, **caractérisé en ce que** l'élément de contact comprend un bouchon conçu pour faire office d'élément d'aspiration afin de fixer solidement le dispositif à la peau.
